# FASCICULE DE BREVET EUROPEEN

(11) **EP 1 491 515 B1**
(45) Date de publication et mention de la délivrance du brevet: **25.07.2007**
(21) Numéro de dépôt: 04291594.2
(22) Date de dépôt: 24.06.2004
(51) Int. Cl.: C03C 17/34

(54) **Matériau souple à contraste optique dans l'infrarouge**
Flexibles Material mit optischem Kontrast im IR-Spektralbereich
Flexible material with optical contrast in the infrared domain

(30) Priorité: 26.06.2003 FR 0307705
(43) Date de publication de la demande: 29.12.2004
(73) Titulaire: Etat-Francais représenté par le Délégué Général pour L'Armement, 94114 Arcueil Cèdex (FR)
(72) Inventeur: Sauques, Laurent, 91160 Ballainvilliers (FR)

(56) Documents cités:
- FR-A- 2 726 545
- US-A- 4 401 690
- US-A- 5 418 640
- US-A- 5 427 835
- US-A- 6 096 964
- US-A1- 2003 054 177
- NIESSNER W ET AL: "Plasmon excitation in vanadium dioxide films" THIN SOLID FILMS, ELSEVIER-SEQUOIA S.A. LAUSANNE, CH, vol. 305, no. 1-2, 1 août 1997 (1997-08-01), pages 61-65, XP004088964 ISSN: 0040-6090

## Description

La présente invention concerne un matériau dont il est possible de moduler, en fonction de la température, les propriétés optiques dans l'infrarouge. Des applications possibles d'un tel matériau concernent notamment la régulation thermique des satellites, les serres agricoles ou les vérandas ou la cosmétologie.

Dans le domaine des serres agricoles, on connaît la demande de brevet US2003/054177. Ce brevet décrit un matériau apte à améliorer le confort à l'intérieur d'une auto ou d'un appartement et consistant en une première couche d'oxyde de vanadium thermochrome et d'une couche d'oxyde de titane destinées à être fixées sur les vitres de l'appartement ou de l'automobile. On connaît aussi le document intitulé « Plasmon excitation in vanadium dioxide films » qui décrit un procédé apte à fixer une couche d'oxyde de vanadium thermochrome sur une plaque de verre, ce procédé mis en oeuvre par un dispositif comportant une enceinte de confinement renfermant une anode, un porte substrat et des moyens de chauffage du substrat, ainsi qu'une cathode connectée à un générateur radiofréquence et connecté à l'extérieur par un circuit d'alimentation en argon, par un circuit d'alimentation en oxygène et par des moyens aptes à réaliser le vide dans l'enceinte, procédé caractérisé en ce qu'il comporte les étapes suivantes : placer une cible à base de vanadium à la cathode et fixer le substrat souple sur le porte substrat, à proximité de l'anode et en regard de la cathode,
- purger l'enceinte de confinement,
- alimenter l'enceinte en argon et en oxygène et actionner les moyens aptes à réaliser le vide dans l'enceinte de telle sorte que la pression à l'intérieur de l'enceinte soit inférieure à 1 Torr, et préférablement comprise entre 1 et 50 mTorr
- chauffer le substrat à une température supérieure à 300°C et inférieure à 500°C
- alimenter la cathode par le générateur radiofréquence afin d'engendrer un dépôt de dioxyde de vanadium thermochrome sur le substrat, et ce jusqu'à obtenir l'épaisseur voulue pour ce dépôt.
Si un tel procédé peut être utilisé à l'échelle d'un parre brise, il ne peut l'être sur des plaques de verre de grandes dimensions ou sur une surface présentant des angles.
En outre, dans le domaine des satellites présentant des formes extérieures complexes, il est impensable de les réaliser en verre recouvert de dioxyde de vanadium.
Enfin, dans le domaine de la cosmétologie, il est difficile d'envisager d'utiliser du verre recouvert par une couche de dioxyde de vanadium.
Le but de l'invention est de résoudre les inconvénients précités en proposant un dispositif apte permettre de moduler les propriétés d'un objet, quelle que soit sa forme et ses dimensions et étant éventuellement apte à être utilisé en cosmétologie.

La solution apportée est un matériau constitué par un substrat recouvert en tout ou partie par une couche comportant du dioxyde de vanadium et caractérisé en ce que le substrat est souple, son épaisseur étant inférieure à 200 µm.
Les dépôts sur support souple s'avèrent intéressant vis-à-vis des propriétés d'adaptabilité liée à la souplesse de l'ensemble. Ainsi, il est préférable d'avoir un ensemble « support + couche active » dont l'épaisseur totale ne dépasse pas 50µm. Les supports inférieurs à 50µm peuvent être de deux types : organique de type kapton ou inorganique de type mica par exemple clivé (muscovite). L'ensemble, du fait de sa souplesse, pourra donc être plaqué et par exemple collé sur des formes particulières planes ou courbes voire angulaires tout en conservant la possibilité de transmettre ou de réfléchir l'infrarouge.

Selon une caractéristique additionnelle, ce matériau est à contraste de propriétés optiques dans l'infrarouge.

Par matériau à contraste de propriétés optiques dans l'infrarouge, il faut entendre un matériau présentant une variation de transmission, de réflexion à une longueur d'onde donnée ou dans une bande de longueurs d'ondes déterminée, principalement dans l'infrarouge, entre la phase semi-conductrice et la phase métallique, cette variation étant d'au moins 10%. Selon une caractéristique particulière, le substrat est souple.

Selon une autre caractéristique particulière, la couche comportant de l'oxyde de vanadium à une épaisseur comprise entre 10 et 1000nm.

Selon une autre caractéristique, le vanadium est dopé par au moins un métal de valence supérieure ou égale à 4 tel par exemple le ruthénium, le molybdène, le tungstène ou le rhénium.

Selon une autre caractéristique, le substrat souple est organique et peut comporter, au moins en partie, du kapton, et peut être constitué par un film de kapton.

Selon une caractéristique additionnelle l'épaisseur de la feuille de kapton est de l'ordre de 50µm.

Selon une autre caractéristique, le substrat souple est inorganique et peut être constitué, au moins en partie par du mica, par exemple par un film de mica dont l'épaisseur est préférablement inférieure à 200 µm.

Selon une caractéristique additionnelle, l'épaisseur de la feuille de kapton ou du film de mica est de l'ordre de 50µm.

Selon une caractéristique particulière permettant son utilisation en cosmétologie, l'épaisseur de substrat est inférieure à 20µm. Ainsi, une fois recouvert par la couche d'oxyde de vanadium, le matériau obtenu peut être finement broyé et mélangé à une crème et ainsi être étendu sur une partie du corps d'une personne afin de la protéger par exemple des rayonnements solaires.

Le dépôt de la couche de vanadium peut être réalisé par tout procédé, incluant notamment les techniques de dépôt sous vide comme l'évaporation par chauffage, par faisceau d'électrons, par dépôt ionique, par rayonnement laser, par l'utilisation de micro ondes, ou par déposition par vapeur chimique (CVD en anglais) à faible pression ou à pression atmosphérique ou par déposition par voie humide.

Toutefois, tous les procédés cités précédemment présentent certains inconvénient tels que l'endommagement du substrat parce qu'il est porté à une température trop élevée ou la présence d'oxyde de vanadium sous une forme cristalline non thermochrome.

Pour solutionner ce problème l'invention consiste aussi en un procédé de fabrication d'un matériau comportant un substrat souple recouvert en tout ou partie par une couche comportant du dioxyde de vanadium thermochrome dopé par du ruthénium, procédé mis en oeuvre par un dispositif comportant une enceinte de confinement renfermant une anode, un porte substrat et des moyens de chauffage du substrat, ainsi qu'une cathode connectée à un générateur radiofréquence et connecté à l'extérieur par un circuit d'alimentation en argon, par un circuit d'alimentation en oxygène et par des moyens aptes à réaliser le vide dans l'enceinte, procédé caractérisé en ce qu'il comporte les étapes suivantes :
- placer une cible à base de vanadium ou d'oxyde de vanadium et du ruthénium à la cathode et fixer le substrat souple sur le porte substrat, à proximité de l'anode et en regard de la cathode,
- purger l'enceinte de confinement,
- alimenter l'enceinte en argon et en oxygène
- chauffer le substrat à une température supérieure à 300°C et inférieure à 500°C
- alimenter la cathode par le générateur radiofréquence afin d'engendrer un dépôt de dioxyde de vanadium thermochrome dopé au ruthénium sur le substrat, et ce jusqu'à obtenir l'épaisseur voulue pour ce dépôt.
Le chauffage du substrat à une température supérieure à 300°C et inférieure à 500°C permet d'obtenir un dépôt de dioxyde de vanadium sous forme thermochrome.

La cible à base de vanadium peut notamment être constituée par de l'oxyde de vanadium VOy, avec y allant de 0 à 2,5 et pouvant être ou non dopé, ou par du vanadium pur ou dopé.

Selon une caractéristique particulière, il comporte une étape consistant à régler l'écartement entre la cathode et l'anode et/ou le substrat de telle sorte que l'espacement entre eux soit compris entre 10⁻¹ et 10⁻² m.

Selon une autre caractéristique, il comporte une étape consistant à associer un métal de valence supérieure à 4 à la cible en vanadium, ce métal pouvant par exemple être du molybdène, du rhénium ou du ruthénium. Plusieurs cibles peuvent donc être placées sur la cathode.

Selon une autre caractéristique, l'étape consistant à purger l'enceinte de confinement consiste à faire le vide avec lesdits des moyens aptes à réaliser le vide dans l'enceinte, et peut consister en outre en une étape de piégeage des impuretés pouvant consister à alimenter un piège à zéolithe en azote liquide.

Selon une caractéristique additionnelle, il consiste en outre à purger les circuits d'alimentation en argon et en oxygène.

Selon une autre caractéristique, le rapport entre le débit massique d'argon et celui d'oxygène pénétrant à l'intérieur de l'enceinte de confinement est supérieur à 50 et préférablement supérieur à 100.

Selon une caractéristique additionnelle, lorsque l'enceinte est alimentée en argon et en oxygène, les moyens aptes à réaliser le vide dans l'enceinte sont actionnés de telle sorte que la pression à l'intérieur de l'enceinte soit inférieure à 1 Torr, et préférablement comprise entre 1 et 50 mTorr

Selon une autre caractéristique, le procédé comporte une étape préalable à celle de l'alimentation de la cathode par le générateur radiofréquence et consistant à purifier la cible de vanadium, cette étape pouvant être mise en oeuvre par un cache amovible apte à être interposé entre la cathode et le substrat et l'étape du procédé consister alors à interposer le cache amovible entre la cathode et le substrat puis à alimenter la cathode par le générateur radiofréquence afin d'engendrer un dépôt de dioxyde de vanadium sur le cache amovible et non pas sur le substrat.

Selon une caractéristique additionnelle, il comporte une étape finale consistant à laisser refroidir le substrat recouvert par une couche de dioxyde de vanadium à une pression comprise entre 1 et 100 mTorr et sous argon.

VO₂ sous sa forme thermochrome est un matériau actif principalement dans l'infrarouge. En effet, il passe d'un état transparent, ou absorbant, à réflecteur à 68°C. De nombreuses applications nécessitent d'avoir une transition en deçà de 68°C. Pour cela on substitue des atomes de vanadium par une entité dopante. Le dopant généralement utilisé est le tungstène connu comme étant le dopant le plus efficace car il permet de baisser la température de transition de VO₂ à raison de-22°C/% atomique. Cependant l'ajout de ce dopant dégrade inéluctablement les contrastes optiques dans l'infrarouge.

L'invention concerne aussi un matériau constitué d'un substrat recouvert en tout ou partie par une couche d'oxyde de vanadium thermochrome et ne comportant pas l'inconvénient susmentionné.

La solution apportée est un matériau constitué d'un substrat recouvert en tout ou partie par une couche d'oxyde de vanadium thermochrome et caractérisée en ce que la couche d'oxyde de vanadium thermochrome est dopée avec du ruthénium.

D'autres avantages et caractéristiques apparaîtront dans la description de modes particuliers de réalisation de l'invention au regard des figures annexées parmi lesquelles :
- La figure 1 présente un exemple de réalisation d'un matériau selon l'invention.

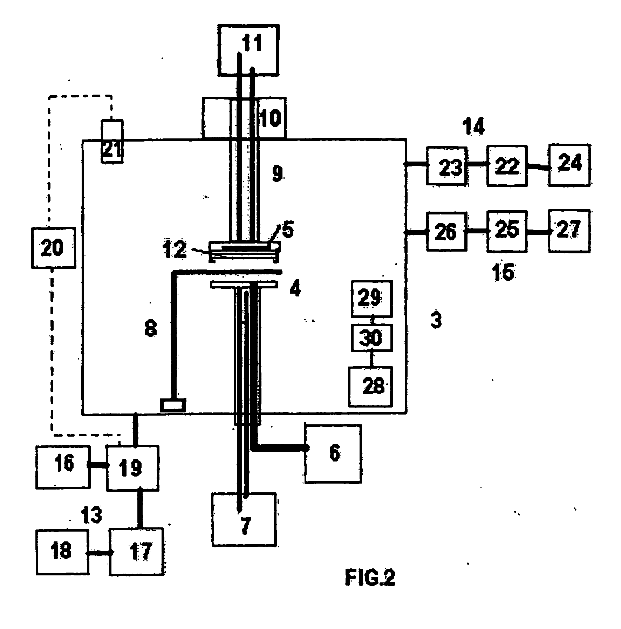

- La figure 2 représente l'enceinte de synthèse.
- La figure 3 représente la transmission collimatée d'une couche de V02 thermochrome sur substrat organique d'épaisseur 50µm (Kapton).
- La figure 4 représente la variation de transmission collimatée en fonction de la température à 4µm d'une couche de VO2 thermochrome sur substrat inorganique d'épaisseur 50µm (mica)
- La courbe de la figure 5 présente la réflexion hémisphérique directionnelle entre 2.5 et 20µm de VO2 thermochrome sur substrat organique de type kapton (50µm d'épaisseur) à 10°C et 90°C.
- La courbe de la figure 6 présente un exemple de l'abaissement de la température de transition en transmission collimatée à 4µm sur support inorganique de type mica.
- Les courbes de la figure 7 qui présentent une comparaison de la transmission, en fonction de la nature du dopant utilisé et de la température de surface, dans l'infrarouge à 3.5µm pour une couche d'oxyde de vanadium de 120 nm d'épaisseur.

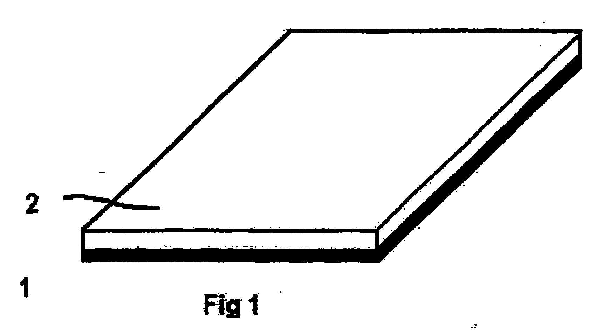

La figure 1 présente un exemple de réalisation d'un matériau selon l'invention. Il est constitué d'un substrat souple en mica d'une épaisseur de l'ordre de 50µm recouvert d'une couche d'oxyde de vanadium thermochrome de formule VO2 dont l'épaisseur lest de 120nm.

Ce matériau a été réalisé avec un dispositif comportant une enceinte de confinement 3 dans laquelle se trouvent une cathode 4 et une anode 5 qui sont positionnée en vis-à-vis à une distance d'environ 4 . 10⁻² m. Cette enceinte de confinement comporte une porte qui permet d'accéder à l'intérieur notamment pour y placer le substrat souple ainsi qu'une cible de vanadium et pour y retirer le matériau selon l'invention.

La cathode 4 est en cuivre et elle est reliée à un générateur radiofréquence 6 accordé à la fréquence de 13,56MHz.

La cathode 4 est refroidie par un circuit de refroidissement 7.

Des moyens 8 amovibles peuvent être interposés entre la cathode 4 et l'anode 5. Leur amovibilité est réalisée par un moteur qui assurent leur pivotement depuis une première position dans laquelle ils sont positionnés entre la cathode 4 et l'anode 5 et obstruent leur vis à vis, et une deuxième position dans laquelle ils n'obstruent plus le vis à vis entre la cathode 4 et l'anode 5. Ce moteur est télécommandé.

L'anode 2 est disposée sur un porte substrat 9 mobile en translation à l'intérieur de l'enceinte de confinement par l'intermédiaire de moyens de réglage 10 constitués par un anneau de serrage. Ce porte substrat 9 est tubulaire et un circuit de refroidissement 11 permet le refroidissement de l'anode 4.

Des résistances chauffantes 12 sont positionnées sur l'anode et un substrat est positionné à 5mm de ces résistances à l'aide de pinces elles-mêmes fixées au porte substrat 9.

Pour ce qui est des gaz contenus dans l'enceinte, cette dernière est connectée à l'extérieure d'une part par des moyens 13 aptes à aspirer les gaz se trouvant à l'intérieure de l'enceinte et d'autre part à un circuit 14 d'alimentation en argon et à un circuit 15 d'alimentation en oxygène.

Les moyens 13 aptes à aspirer les gaz se trouvant à l'intérieur de l'enceinte sont constitués par des moyens de pompage primaire constitués par une pompe à palette 16 et par des moyens de pompage secondaire constitués par une pompe turbo 17 et une pompe à palette 18.

Une vanne de régulation 19 est positionnée en amont des moyens de pompage secondaire et reliée à des moyens de contrôle commande 20 de la pression eux-mêmes reliés à un capteur de pression 21.

Le circuit d'alimentation en argon 14 comporte une vanne de régulation 22 un débitmètre 23 relié à une bouteille 24 d'argon sous pression.

Le circuit d'alimentation en oxygène 15 comporte une vanne de régulation 25, un débitmètre 26 relié à une bouteille 27 d'oxygène sous pression.
La régulation des vannes de régulation 22 et 25 est du type maître esclave afin de conserver un ratio toujours constant entre le débit massique d'argon et celui d'oxygène.

Un piège à zéolithe 28 est disposé à l'intérieur de l'enceinte de confinement 3 et relié à une réserve d'azote liquide 29 dont la sortie est obturée par une vanne qui peut être télécommandée à distance.

Le procédé de fabrication d'un matériau constitué par un substrat souple recouvert en tout ou partie par une couche comportant de l'oxyde de vanadium thermochrome de formule V02 est le suivant :
La porte de l'enceinte est ouverte et un substrat constitué par une feuille de mica de 50µm est fixé sur le porte substrat 9 par l'intermédiaire des dites pinces Le porte substrat est constitué d'une zone évidée permettant un chauffage radiatif et non conductif. Une plaquette de vanadium pur à 99,99% est déposée sur la cathode 4 ; l'ajout de pastilles d'un métal de valence supérieure ou égale à 4, tel le ruthénium, le molybdène déposées sur la plaquette de vanadium étant justifié pour l'abaissement de la température de transition.

La purge de l'enceinte de confinement est ensuite effectuée. Pour cela un vide est réalisé dans l'enceinte avec une valeur de consigne à 10⁻⁷ Torr.

Lorsque cette valeur de vide est atteinte, l'ouverture de la vanne située en sortie du réservoir d'azote liquide est télécommandée, permettant alors à de l'azote liquide de s'écouler dans le piège de zéolithe jusqu'à remplir ce dernier. La fermeture de ladite vanne est ensuite télécommandée. L'azote liquide à deux fonctions principales, la première étant de refroidir l'intérieur de l'enceinte, donc de diminuer encore la pression dans l'enceinte et la seconde de piéger des impuretés gazeuses qui pourraient demeurer à l'intérieur de l'enceinte.

Les conduits d'arrivée de gaz à l'intérieur de l'enceinte sont ensuite purgés par ouverture forcée des vannes de régulation 22 et 25.

Une phase de purification de la cible de vanadium est ensuite effectuée. Elle consiste à positionner le cache amovible entre la cathode et le substrat puis à alimenter l'enceinte en gaz argon par ouverture de la vanne de régulation 22 avec une valeur de consigne du débitmètre massique 23 à 80 . 10⁻⁶ m³ standards par minute, puis alimentation de la cathode par le générateur radiofréquence de façon à générer un champ électrique entre la cathode et l'anode. Cette phase est réalisée pendant 30minutes avec une puissance du générateur radiofréquence de 250W et une valeur de consigne de la pression à l'intérieur de l'enceinte de 15mTorr.

A l'issue de cette phase de purification, un vide est réalisé dans l'enceinte avec une valeur de consigne à 10⁻⁷ Torr.

Lorsque cette valeur de vide est atteinte, les moyens de régulation des vannes 22 et 25 sont mis en fonctionnement de type maître/esclave avec une valeur de consigne de 80 . 10⁻⁶ m³ par minute pour l'argon et un ratio de 111 entre le débit massique d'argon et celui d'oxygène, le débit d'oxygène correspondant étant alors de 0,72 . 10⁻⁶ m³ par minute. Par ailleurs, les moyens de chauffage du substrat sont mis en fonctionnement de façon à ce que le substrat atteigne progressivement une température d'environ 410°C, puis cette température et maintenue pendant une phase dite de stabilisation pendant environ 30 minutes. De plus la valeur de la consigne de pression à l'intérieur de l'enceinte est fixée à 15mtorr. Lorsque cette pression est atteinte le générateur radiofréquence est mis en fonctionnement pour alimenter la cathode puis le moteur associé au cache amovible est télécommandé afin de supprimer son interposition entre la cathode et le substrat qui se font alors face et le dépôt de dioxyde de vanadium sur le substrat commencent, ce dioxyde de vanadium étant, compte tenu du procédé ainsi réalisé, sous une forme cristalline thermochrome.

Lorsque l'épaisseur de la couche de dioxyde de vanadium recouvrant le substrat est suffisante, le générateur de radiofréquence est arrêté ainsi que les moyens de chauffage du substrat et l'alimentation en oxygène et le substrat est laissé à refroidir sous argon à une pression de consigne de 50mTorr pendant 3 heures.

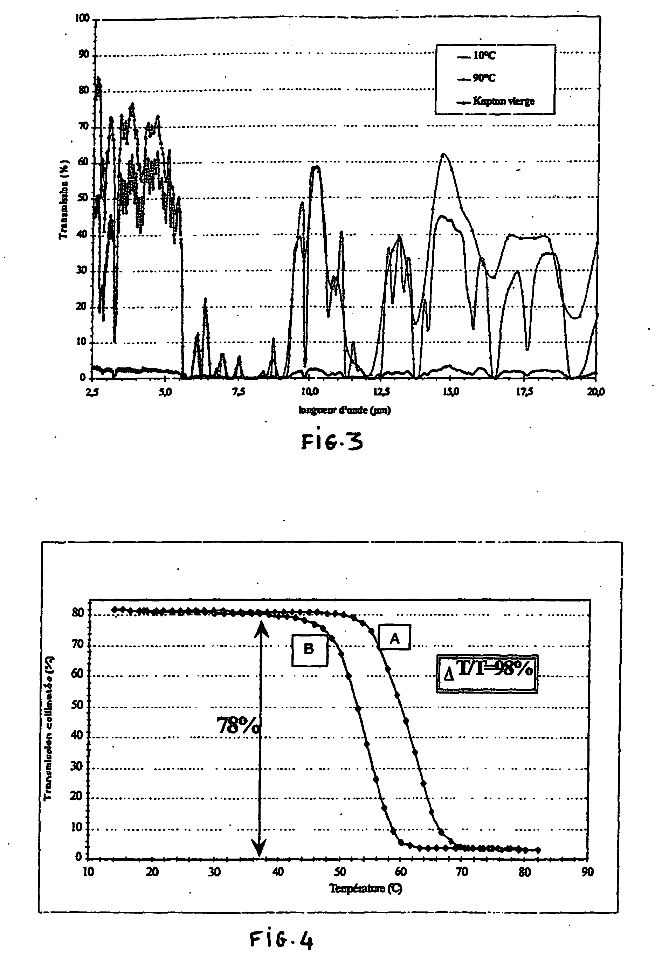

La figures 3 montrent la courbe représentative de la transmission collimatée d'un matériau constitué par un substrat souple organique (kapton d'épaisseur 50µm) recouvert par une couche de dioxyde de vanadium thermochrome d'épaisseur 120nm). A 10°C, l'ensemble est transparent puisque l'allure du spectre suit l'allure du substrat souple de la couche. A 90°C, la transmission a chuté pour ne valoir que quelques pourcents. L'ensemble substrat+couche de dioxyde de vanadium thermochrome n'est donc plus transparent.

La figure 4 montre la courbe représentative de la transmission collimatée à 4µm, d'une couche de VO2 thermochrome de 125 nm d'épaisseur sur un substrat inorganique de type Mica d'épaisseur 50µm, en fonction de la température Les courbes A et B de la figure 4 ont été obtenues avec un matériau réalisé avec un procédé selon l'invention. La transmission à 4 µm est comprise en phase d'échauffement (partie A) du matériau entre 80 et 82% depuis la température ambiante jusqu'à 52°C ou la transmission n'est plus constante. On constate qu'entre 52°C et 68°C, la transmission décroît avec l'augmentation de la température depuis une valeur de 81% à 52°C jusqu'à une valeur de 3% à 68°C et plus, cette température étant température de stabilisation à l'échauffement.

En phase de refroidissement (partie B), la transmission collimatée est constante pour des températures supérieures ou égales à 60°C. On constate qu'entre 60°C et 44°C, la transmission croit avec la température depuis une valeur de 3% à 60°c jusqu'à une valeur de 81 % à 44°C et moins, cette température étant appelée température de stabilisation au refroidissement.

La température de transition étant définie à mi-largeur de cycle, elle est de 62°C lors du chauffage et de 55°C en refroidissement.

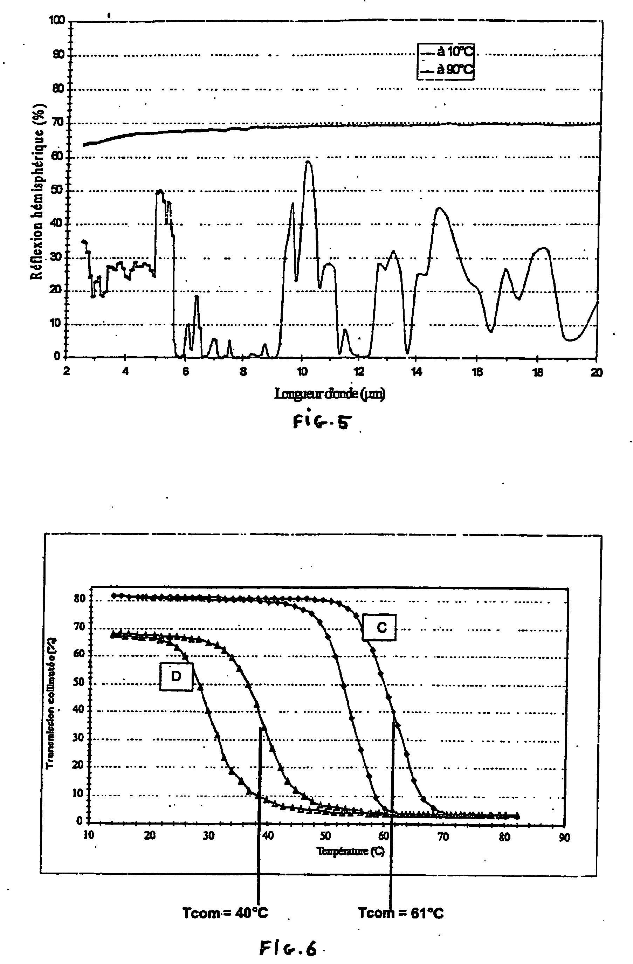

La figure 5 montre la courbe représentative de la réflexion hémisphérique directionnelle d'un matériau constitué par un substrat souple organique (kapton d'épaisseur 50µm) recouvert par une couche de dioxyde de vanadium thermochrome d'épaisseur 120nm. A 10°C, la couche est peu réflectrice en fonction de la longueur d'onde et dépendante de celle-ci. V02 thermochrome étant transparent, nous observons donc la réflexion du substrat organique. A 90°C, la couche devient réflectrice ; une valeur proche de 70% est ainsi obtenue à partir de 8 µm. Cette réponse est quasiment plate et indépendante de la nature du substrat.

La figure 6 montre les courbes représentatives de la transmission collimatée à 4µm, en fonction de la température, de deux réalisations suivant le procédé de réalisation. La température de commutation a nettement baissé passant de 61°C à 40°C. La réalisation ayant une température de transition (ou de commutation) à 40°C (partie C), a une valeur de transmission égale à 68% de 10 à 28°C. Une baisse de transmission, de 28°C à 48°C, est observée pour se stabiliser à cette température et au-delà, à hauteur de 3%. Lors du refroidissement (partie D), de 50°C et au-dessus, la transmission est constante et égale à 3%. De 50°C jusqu'à 24°C, la transmission augmente pour atteindre 68% à 24°C. En dessous de cette température, la transmission, la transmission demeure constante et égale à 68%.

Selon le métal de dopage utilisé la température de commutation selon l'invention est comprise entre -5°C et 70°C.

Dans le cas de serres agricoles comportant par exemple une enveloppe en verre, un substrat selon l'invention peut être utilisé afin de recouvrir cette enveloppe. Du fait de la très faible épaisseur de ce substrat, le rayonnement solaire peut le traverser et pénétrer dans la serre. Cependant lorsqu'il atteint la température de commutation qui est, notamment, fonction de la température à l'intérieur de la serre, sa transmission décroît ce qui limite la pénétration du rayonnement solaire à l'intérieur de la serre, qui, de ce fait ne s'échauffe plus. Ce revêtement selon l'invention permet donc de limiter la température maximale à l'intérieur de la serre.

Par ailleurs, d'autres applications concernent l'utilisation d'un matériau selon l'invention constitué d'un substrat recouvert en tout ou partie par une couche comportant de l'oxyde de vanadium sur des parties tièdes ou chaudes ou susceptibles de s'échauffer d'un objet.

Selon une caractéristique particulièrement intéressante, la couche d'oxyde de vanadium thermochrome est dopée avec du ruthénium.

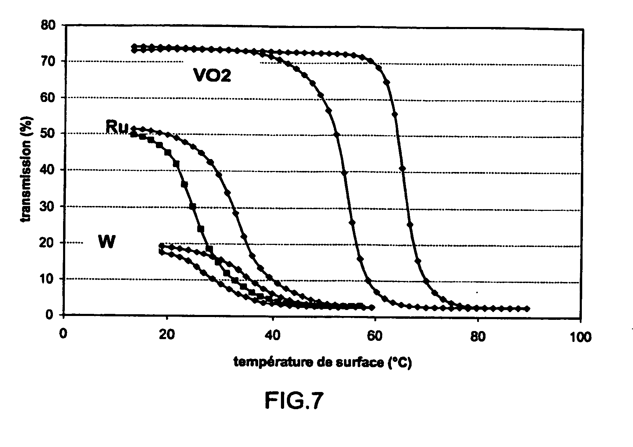

Les courbes de la figure 7 présentent une comparaison en transmission dans l'infrarouge à 3.5µm pour une couche d'oxyde de vanadium de 120 nm d'épaisseur, et respectivement pour de l'oxyde de vanadium pur (VO2), pour du tungstène (W) et pour du ruthénium (Ru), le dopage au ruthénium s'avère meilleur que celui au tungstène. En effet à température de transition identique, le contraste en transmission est supérieur à celui obtenu avec du tungstène. Ce résultat a été obtenu sur support souple et sur support rigide, notamment sur une vitre de plusieurs millimètres d'épaisseur. En outre, la substitution s'effectue plus facilement car il y a une meilleure compatibilité au niveau des rayons atomiques entre le vanadium et le ruthénium (vanadium 134 pm, ruthénium 135 pm et tungstène 141 pm).

## Revendications

1. Matériau constitué par un substrat (2) recouvert en tout ou partie par une couche (1) comportant du dioxyde de vanadium thermochrome, **caractérisé en ce que** le substrat (2) est souple et son épaisseur est inférieure à 200 µm.

2. Matériau selon la revendication 1, **caractérisé en ce qu'**il est à contraste d'émission.

3. Matériau selon l'une quelconque des revendications 1 et 2, **caractérisé en ce que** la couche comportant de l'oxyde de vanadium à une épaisseur comprise entre 30 et 200nm.

4. Matériau selon l'une quelconque des revendications 1 à 3, **caractérisé en ce que** le substrat souple comporte, au moins en partie, du kapton.

5. Matériau selon l'une quelconque des revendications 1 à 3, **caractérisé en ce que** le substrat souple comporte, au moins en partie, du mica.

6. Matériau selon l'une quelconque des revendications 4 et 5, **caractérisé en ce que**, l'épaisseur du substrat est de l'ordre de 50µm.

7. Matériau selon l'une quelconque des revendications 1 à 6, **caractérisé en ce que** le vanadium est dopé par au moins un métal de valence supérieure ou égale à 4.

8. Matériau selon la revendication 7, **caractérisé en ce que** le vanadium est dopé par du ruthénium.

9. Matériau selon la revendication 8, **caractérisé en ce que** le support souple d'épaisseur inférieure à 200 µm est substitué par un support rigide.

10. Procédé de fabrication d'un matériau selon l'une quelconque des revendications 7 à 9 mis en oeuvre par un dispositif comportant une enceinte de confinement renfermant une anode, un porte substrat et des moyens de chauffage du substrat, ainsi qu'une cathode connectée à un générateur radiofréquence et connecté à l'extérieur par un circuit d'alimentation en argon, par un circuit d'alimentation en oxygène et par des moyens aptes à réaliser le vide dans l'enceinte, procédé **caractérisé en ce qu'**il comporte les étapes suivantes :
- placer une cible à base de vanadium et du ruthénium à la cathode et fixer le substrat sur le porte substrat, à proximité de l'anode et en regard de la cathode,
- purger l'enceinte de confinement,
- alimenter l'enceinte en argon et en oxygène et actionner les moyens aptes à réaliser le vide dans l'enceinte de telle sorte que la pression à l'intérieur de l'enceinte soit inférieure à 1 Torr, et préférablement comprise entre 1 et 50 mTorr
- chauffer le substrat à une température supérieure à 300°C et inférieure à 500°C
- alimenter la cathode par le générateur radiofréquence afin d'engendrer un dépôt de dioxyde de vanadium thermochrome dopé au ruthénium sur le substrat, et ce jusqu'à obtenir l'épaisseur voulue pour ce dépôt.

11. Procédé selon la revendication 10, **caractérisé en ce qu'** il comporte une étape consistant à régler l'écartement entre la cathode et l'anode et/ou le substrat de telle sorte que l'espacement entre eux soit compris entre 10⁻¹ et 10⁻² m.

12. Procédé selon la revendication 11, **caractérisé en ce que** l'étape consistant à purger l'enceinte de confinement consiste en outre en une étape de piégeage des impuretés pouvant consister à alimenter un piège à zéolithe en azote liquide.

13. Procédé selon l'une quelconque des revendications 11 et 12, **caractérisé en ce que** l'étape consistant à purger l'enceinte de confinement consiste en outre à purger les circuits d'alimentation en argon et en oxygène.

14. Procédé selon l'une quelconque des revendications 10 à 13, **caractérisé en ce que** le rapport entre le débit massique d'argon et celui d'oxygène pénétrant à l'intérieur de l'enceinte de confinement est supérieur à 50 et préférablement supérieur à 100.

15. Procédé selon l'une quelconque des revendications 10 à 14, **caractérisé en ce qu'**il comporte une étape préalable à celle de l'alimentation de la cathode par le générateur radiofréquence et consistant à purifier la cible de vanadium.

16. Procédé selon la revendication 15, **caractérisé en ce que** l'étape consistant à purifier la cible de vanadium implique la mise en oeuvre par un cache amovible apte à être interposé entre la cathode et le substrat et l'étape du procédé consiste alors à interposer le cache amovible entre la cathode et le substrat puis à alimenter la cathode par le générateur radiofréquence afin d'engendrer un dépôt de dioxyde de vanadium sur le cache amovible et non pas sur le substrat.

17. Procédé selon l'une quelconque des revendications 10 à 16, **caractérisé en ce qu'**il comporte une étape finale consistant à laisser refroidir le substrat recouvert par une couche de dioxyde de vanadium à une pression comprise entre 1 et 100 mTorr et sous argon.

18. Application d'un matériau selon l'une quelconque des revendications 1 à 9 sur tout ou partie des parties tièdes ou chaudes ou susceptibles de s'échauffer d'un objet.

## Claims

1. Material constituted by a substrate (2) covered wholly or partially by a layer (1) comprising thermochromic vanadium dioxide, **characterised in that** the substrate (2) is flexible and its thickness is less than 200 µm.

2. Material according to claim 1, **characterised in that** it has emission contrast.

3. Material according to either claim 1 or claim 2, **characterised in that** the layer comprising vanadium oxide has a thickness of from 30 to 200 nm.

4. Material according to any one of claims 1 to 3, **characterised in that** the flexible substrate is constituted, at least partially, by Kapton.

5. Material according to any one of claims 1 to 3, **characterised in that** the flexible substrate is constituted, at least partially, by mica.

6. Material according to either claim 4 or claim 5, **characterised in that** the thickness of the substrate is of the order of 50 µm.

7. Material according to any one of claims 1 to 6, **characterised in that** the vanadium is doped with at least one metal having a valency greater than or equal to 4.

8. Material according to claim 7, **characterised in that** the vanadium is doped with ruthenium.

9. Material according to claim 8, **characterised in that** the flexible support having a thickness of less than 200 µm is replaced by a rigid support.

10. Method for the production of a material according to any one of claims 7 to 9, carried out by a device comprising a containment which contains an anode, a substrate support and means for heating the substrate, as well as a cathode connected to a radiofrequency generator, and which is connected to the outside by way of an argon supply circuit, by way of an oxygen supply circuit and by way of means capable of producing a vacuum in the containment, which method is **characterised in that** it comprises the following steps:
- a vanadium- and ruthenium-based target is placed at the cathode and the substrate is fixed to the substrate support, close to the anode and facing the cathode,
- the containment is purged,
- the containment is supplied with argon and with oxygen, and the means capable of producing a vacuum in the containment are operated so that the pressure inside the containment is below 1 torr and preferably from 1 to 50 mtorr,
- the substrate is heated to a temperature greater than 300°C and less than 500°C,
- power is supplied to the cathode by the radiofrequency generator in order to generate a deposit of ruthenium doped thermochromic vanadium dioxide on the substrate, until the desired thickness of the deposit is obtained.

11. Method according to claim 10, **characterised in that** it comprises a step in which the spacing between the cathode and the anode and/or the substrate is adjusted so that the spacing between them is between 10⁻¹ and 10⁻² m.

12. Method according to claim 11, **characterised in that** the step in which the containment is purged further comprises a step of trapping impurities, which step can consist in supplying liquid nitrogen to a zeolite trap.

13. Method according to either claim 11 or claim 12, **characterised in that** the step in which the containment is purged further comprises purging the argon and oxygen supply circuits.

14. Method according to any one of claims 10 to 13, **characterised in that** the ratio between the mass flow rate of argon and that of oxygen entering the containment is greater than 50 and preferably greater than 100.

15. Method according to any one of claims 10 to 14, **characterised in that** it comprises a step that precedes the step of supplying power to the cathode by means of the radiofrequency generator and that comprises purifying the vanadium target.

16. Method according to claim 15, **characterised in that** the step in which the vanadium target is purified is carried out by means of a removable screen capable of being interposed between the cathode and the substrate, and the step of the method then comprises interposing the removable screen between the cathode and the substrate and then supplying power to the cathode by means of the radiofrequency generator in order to generate a deposit of vanadium dioxide on the removable screen and not on the substrate.

17. Method according to any one of claims 10 to 16, **characterised in that** it comprises a final step in which the substrate covered by a layer of vanadium dioxide is allowed to cool at a pressure of from 1 to 100 mtorr and under argon.

18. Application of a material according to any one of claims 1 to 9 to all or part of the parts of an object that are warm or hot or liable to become warm or hot.

## Patentansprüche

1. Material, das aus einem Substrat (2) besteht, das vollständig oder teilweise mit einer thermochromes Vanadiumdioxid enthaltenden Schicht (1) überzogen ist, **dadurch gekennzeichnet, dass** das Substrat (2) biegsam ist und seine Dicke weniger als 200 µm beträgt.

2. Material nach Anspruch 1, **dadurch gekennzeichnet, dass** es strahlungskontrastierend ist.

3. Material nach einem der Ansprüche 1 und 2, **dadurch gekennzeichnet, dass** die thermochromes Vanadiumdioxid enthaltende Schicht eine Dicke zwischen 30 und 200 nm aufweist.

4. Material nach einem der Ansprüche 1 bis 3, **dadurch gekennzeichnet, dass** das biegsame Substrat mindestens teilweise Kapton aufweist.

5. Material nach einem der Ansprüche 1 bis 3, **dadurch gekennzeichnet, dass** das biegsame Substrat mindestens teilweise Mika aufweist.

6. Material nach einem der Ansprüche 4 und 5, **dadurch gekennzeichnet, dass** die Dicke des Substrats in der Größenordnung von 50 µm beträgt.

7. Material nach einem der Ansprüche 1 bis 6, **dadurch gekennzeichnet, dass** das Vanadium mit mindestens einem Metall dotiert ist, dessen Wertigkeit größer als oder gleich 4 ist.

8. Material nach Anspruch 7, **dadurch gekennzeichnet, dass** das Vanadium mit Ruthenium dotiert ist.

9. Material nach Anspruch 8, **dadurch gekennzeichnet, dass** der biegsame Träger mit einer Dicke unter 200 µm durch einen steifen Träger ersetzt ist.

10. Verfahren zur Herstellung eines Material nach einem der Ansprüche 7 bis 9, das mit einer Vorrichtung durchgeführt wird, die eine geschlossene Kammer aufweist, welche eine Anode, einen Substratsträger und Mittel zum Heizen des Substrats sowie eine mit einem Hochfrequenzgenerator verbundene Kathode einschließt und nach außen durch einen Argonspeisekreis, einen Sauerstoffspeisekreis und Mittel verbunden ist, die das Vakuum in der Kammer erzeugen können, **dadurch gekennzeichnet, dass** es folgende Schritte umfasst:
- Anbringen eines Targets auf Vanadium und Ruthenium an der Kathode und Befestigung des Substrats am Substratträger in der Nähe der Anode und gegenüber der Kathode,
- Spülen der geschlossenen Kammer,
- Speisen der Kammer mit Argon und Sauerstoff und Betätigen der Mittel zum Erzeugen von Vakuum in der Kammer, so dass der Druck innerhalb der Kammer unter 1 Torr und vorzugsweise zwischen 1 und 50 Torr liegt,
- Erhitzen des Substrats auf eine Temperatur über 300°C und unter 500°C,
- Speisen der Kathode durch den Hochfrequenzgenerator, um auf dem Substrat eine Abscheideschicht von thermochromem Vanadiumdioxid dotiert mit Ruthenium zu erzeugen, und zwar bis eine gewünschte Dicke für diese Abscheideschicht erhalten wird.

11. Verfahren nach Anspruch 10, **dadurch gekennzeichnet, dass** es einen Schritt des Einstellens des Abstands zwischen der Kathode und der Anode und/oder dem Substrat umfasst, so dass der Abstand zwischen ihnen zwischen 10⁻¹ und 10⁻² m beträgt.

12. Verfahren nach Anspruch 11, **dadurch gekennzeichnet, dass** der Schritt des Spülens der geschlossenen Kammer zudem einen Schritt des Einfangens der Verunreinigungen umfasst, der darin bestehen kann, eine Zeolithfalle mit Flüssigstickstoff zu speisen.

13. Verfahren nach einem der Ansprüche 11 und 12, **dadurch gekennzeichnet, dass** der Schritt des Spülens der geschlossenen Kammer zudem darin besteht, die Argon- und Sauerstoffspeisekreise zu spülen.

14. Verfahren nach einem der Ansprüche 10 bis 13, **dadurch gekennzeichnet, dass** das Verhältnis zwischen den innerhalb der geschlossenen Kammer durchdringenden Massenflüssen von Argon und von Sauerstoff größer als 50 und vorzugsweise größer als 100 ist.

15. Verfahren nach einem der Ansprüche 10 bis 14, **dadurch gekennzeichnet, dass** es vor dem Schritt des Speisens der Kathode durch den Hochfrequenzgenerator einen Schritt des Reinigens des Vanadiumtargets umfasst.

16. Verfahren nach Anspruch 15, **dadurch gekennzeichnet, dass** der Schritt des Reinigens des Vanadiumtargets die Durchführung durch eine abnehmbare Abdeckvorrichtung voraussetzt, die zwischen die Kathode und das Substrat eingesetzt werden kann, und dass der Schritt des Verfahrens dann darin besteht, die abnehmbare Abdeckvorrichtung zwischen die Kathode und das Substrat zu setzen und dann die Kathode über den Hochfrequenzgenerator zu speisen, um das Abscheiden einer Vanadiumdioxidschicht auf der abnehmbaren Abdeckvorrichtung und nicht auf dem Substrat zu erzeugen.

17. Verfahren nach einem der Ansprüche 10 bis 16, **dadurch gekennzeichnet, dass** es einen abschließenden Schritt umfasst, der darin besteht, das durch eine Vanadiumdioxidschicht überzogene Substrat bei einem Druck zwischen 1 und 100 mTorr und unter Argon abkühlen zu lassen.

18. Anwendung eines Materials nach einem der Ansprüche 1 bis 9 auf alle oder einen Teil der lauwarmen oder heißen oder erhitzbaren Teile eines Gegenstands.
